# EUROPÄISCHE PATENTSCHRIFT

(11) **EP 2 300 818 B1**
(45) Veröffentlichungstag und Bekanntmachung des Hinweises auf die Patenterteilung: **23.01.2013**
(21) Anmeldenummer: 09749641.8
(22) Anmeldetag: 22.05.2009
(51) Int. Cl.: G01N 33/15, B01F 1/00

(54) **VORRICHTUNG ZUR AUFNAHME EINES FESTSTOFFES IN EINER MESSZELLE**
DEVICE FOR RECEIVING A SOLID IN A MEASURING CELL
DISPOSITIF DE LOGEMENT D'UN SOLIDE DANS UNE CELLULE DE MESURE

(30) Priorität: 23.05.2008 DE 102008024840
(43) Veröffentlichungstag der Anmeldung: 30.03.2011
(73) Patentinhaber: Merck Patent GmbH, 64293 Darmstadt (DE)
(72) Erfinder: LANGE, Thomas, 07749 Jena (DE); PARTHEIL, Anette, 64372 Ober-Ramstadt (DE)
(74) Vertreter: Katscher Habermann Patentanwälte
(86) Internationale Anmeldenummer: PCT/EP2009/003627
(87) Internationale Veröffentlichungsnummer: WO 2009/141151

(56) Entgegenhaltungen:
- WO-A2-01/38850
- US-A- 3 791 222
- US-A- 3 802 272
- US-A- 4 856 909
- US-A- 5 142 920

## Beschreibung

Die Erfindung betrifft eine Vorrichtung zur Aufnahme eines Feststoffes in einer Messzelle, bei welcher die Freisetzung des Feststoffes in einem durch die Messzelle hindurch fließenden Lösungsmedium ermittelt werden kann.

Um die Eigenschaften eines Wirkstoffs und insbesondere dessen Freisetzungsrate bzw. Auflösung in flüssigen Medien zu untersuchen, ist es bekannt, den Wirkstoff in fester Form in eine geeignete Messzelle mit einem Lösungsmedium zu geben und die freigesetzte Menge des Wirkstoffs in Abhängigkeit von der Zeit zu erfassen. Da die Freisetzung eines Wirkstoffs sowohl bei der Herstellung als auch bei der Dosierung und Verabreichung von pharmazeutischen Präparaten berücksichtigt werden muss und oftmals von großer Bedeutung ist, wurden verschiedene Messzellen und Messverfahren entwickelt, mittels derer möglichst reproduzierbare Messergebnisse ermittelt werden können, um ein Vergleichen von Messergebnissen aus unterschiedlichen Versuchsreihen und aus unterschiedlichen Quellen zu ermöglichen. Die verwendeten Messvorrichtungen sollen zweckmäßigerweise sowohl einfach zu bedienen sein und gleichzeitig zuverlässige und präzise Messergebnisse ermöglichen.

Die Freisetzungsrate eines Wirkstoffs, der in Form eines Pulvers, eines Granulats oder einer Tablette vorliegt, kann mit beispielsweise in EUROPEAN PHARMACOPOEIA (2.9.3. "dissolution test for solid dosage forms" & 2.9.43. "apparent dissolution"), USP <711> und JP XIV General Tests 15 beschriebenen Durchfluss-Messzellen ermittelt werden. Die dort beschriebenen Durchfluss-Messzellen weisen einen näherungsweise säulenförmigen Messraum auf, bei welchem vorzugsweise von unten das gewählte Lösungsmedium zugeführt und durch den Messraum hindurch gepumpt wird, um an seinem oberen Ende aus dem Messraum abgeführt, um einer Messeinrichtung zugeführt zu werden. Im Inneren des Messraums befindet sich üblicherweise eine beispielsweise gitterförmige oder schalenförmige Aufnahmevorrichtung für eine Tablette oder ein Granulat mit dem darin enthaltenen Wirkstoff. Die Aufnahmevorrichtung ist dauerhaft in dem Messraum befestigt bzw. gelagert, um auch über viele Messreihen hin eine gleichbleibende Geometrie und damit vergleichbare Messbedingungen gewährleisten zu können. Eine in der Praxis häufig verwendete Messzelle dieser Bauart wird beispielsweise in US 5,142,920 A beschrieben.

Um den Wirkstoff in Tablettenform oder in Granulatform bringen zu können, so dass Messungen mit einer vorangehend beschriebenen Durchfluss-Messzelle ermöglicht werden, muss der interessierende Wirkstoff regelmäßig mit einem geeigneten Hilfsstoff vermengt werden, welches die für die Herstellung von Tabletten bzw. von Granulat erforderlichen physikalischen und insbesondere mechanischen Eigenschaften aufweist. Die in einer Durchflusszelle gemessene Freisetzungsrate des Wirkstoffs ist demzufolge ganz erheblich von dem verwendeten Hilfsstoff abhängig. Es hat sich zusätzlich gezeigt, dass die in der Praxis messbare Freisetzungsrate des Wirkstoffs dabei nicht nur von der Art bzw. der Zusammensetzung des Hilfsstoffs abhängig ist, sondern auch von weiteren Eigenschaften abhängt, wie beispielsweise von dem bei der Tablettenherstellung angewandten Druck oder von dem Verhältnis von Wirkstoff zu Hilfsstoff. Um verschiedene Messergebnisse vergleichen zu können, ist es deshalb trotz der Verwendung weitestgehend einheitlicher Durchfluss-Messzellen erforderlich, viele Parameter zu erfassen und bei der Auswertung bzw. bei einem Vergleich der Messergebnisse für die ermittelte Freisetzungsrate zu berücksichtigen.

Um die innere Freisetzungsrate eines Wirkstoffs, welche die Freisetzung einer reinen festen Substanz mit idealerweise zu vernachlässigenden Porosität beschreibt, einfach und zuverlässig messen zu können und einen Vergleich der erhaltenen Messwerte für die innere Freisetzungsrate zu ermöglichen, wurden verschiedene Verfahren und Vorrichtungen zur Messung der inneren Freisetzungsrate entwickelt. Bei einem weithin bekannten und ebenfalls in EUROPEAN PHARMACOPOEIA (2.2.29. "intrinsic dissolution") monografierten Messverfahren wird ein scheibenförmiger Pressling aus einem Wirkstoff hergestellt und derart in einem Messkopf angeordnet, dass ausschließlich eine kreisförmige Außenfläche des Presslings von außen zugänglich ist. Der Messkopf wird dann an einem drehbar gelagerten Stab in einen Vorratsbehälter mit einem Lösungsmedium eingebracht, so dass die von außen zugängliche Außenseite des Presslings nach unten in Richtung des Bodens des Vorratsbehälters gerichtet ist. Der Messkopf rotiert während der Messdauer, um einerseits eine gleichmäßige Durchmischung des Lösungsmediums zu bewirken und andererseits zu verhindern, dass sich Gasbläschen an der exponierten Oberfläche des Presslings ansammeln, welche die Freisetzung und Auflösung des Wirkstoffs beeinträchtigen und die Messergebnisse verfälschen könnten.

Eine Weiterentwicklung dieser Messzelle wird beispielsweise in US 6,497,157 B1 beschrieben. Bei der dort beschriebenen Messzelle ist der Pressling mit der exponierten Oberfläche nach oben stationär am Boden eines mit einem Lösungsmittel gefüllten Behälters angeordnet. Oberhalb des Presslings ist ein Rührer angeordnet, der während des Messvorgangs das Lösungsmittel in dem Vorratsbehälter durchmischt und lokale Konzentrationsveränderungen oder andere Inhomogenitäten verhindern soll.

Den bekannten derartigen Messvorrichtungen ist gemeinsam, dass zwar die innere Freisetzungsrate eines Presslings aus einem Wirkstoff gemessen werden kann und ein oftmals unerwünschter Einfluss des für die Tablettenherstellung erforderlichen Bindemittels vermieden werden kann, dass aber derartige Messvorrichtungen üblicherweise keine kontinuierliche Auswertung ermöglichen, sondern erst nach Ablauf einer vorgegebenen Messdauer die in dem Lösungsmedium gelöste Menge des freigesetzten Wirkstoffs ermittelt werden kann. Da die monografierten bzw. in der Praxis häufig verwendeten Messvorrichtungen oftmals Vorratsbehälter für das Lösungsmedium mit einem Füllvolumen von mehr als 200 ml und teilweise bis zu 4000 ml aufweisen, sind die mit diesen Messvorrichtungen durchführbaren Messungen material- und kostenaufwendig. Das bei einer Messung verwendete Volumen des Lösungsmediums kann jedoch nicht beliebig klein vorgegeben werden, da sich in diesem Fall bereits nach kurzer Zeit auf Grund nicht gegebener Sinkbedingungen das Auflösungsverhalten des Wirkstoffs durch den bereits gelösten Wirkstoff beeinflusst wird.

In WO2006/108908 A1 wird neben einigen der vorangehend genannten Messvorrichtungen eine weitere Messvorrichtung beschrieben, die als Durchfluss-Messzelle ausgestaltet ist und zur Messung der Freisetzungsrate verwendet werden kann. Das Lösungsmedium wird dabei als dünner Film mit laminarer oder turbulenter Strömung an der Oberfläche eines Presslings bzw. eines verfestigten Wirkstoffs vorbeigeführt. Die während eines Messvorgangs erhaltenen Messergebnisse können allerdings nicht ohne Weiteres mit denjenigen Messergebnissen für denselben Feststoff verglichen werden, die mit anderen Messvorrichtungen ermittelt werden, da sich die äußeren Randbedingungen wie beispielsweise die Strömungsführung des Lösungsmediums im Bereich um den verfestigten Wirkstoff merklich gegenüber denjenigen anderer Messvorrichtungen unterscheiden.

Aufgabe der vorliegenden Erfindung ist es demzufolge, eine Vorrichtung zur Durchführung von Messungen der inneren Freisetzungsrate eines Wirkstoffs so auszugestalten, dass mit einfachen Mitteln präzise und vergleichbare Messergebnisse zur Bestimmung der inneren Freisetzungsrate ermittelt werden können.

Diese Aufgabe wird erfindungsgemäß dadurch gelöst, dass die Vorrichtung zur Aufnahme eines Feststoffes in einer eingangs genannten Messzelle einen in die Messzelle einbringbaren Einsatz mit einer Ausnehmung zur Aufnahme eines Presslings aufweist, wobei der Einsatz ein Gehäuse mit einer durchgehenden Bohrung, eine in der Bohrung aufnehmbare Hülse zur Aufnahme des Presslings und eine Verschlusseinrichtung aufweist.

Die äußeren Abmessungen des Gehäuses sind dabei zweckmäßigerweise an die Abmessungen eines Messraums in der Messzelle angepasst. Der Außendurchmesser der Hülse ist an die durchgehende Bohrung in dem Gehäuse angepasst, so dass die Hülse im Wesentlichen passgenau in der Bohrung angeordnet werden kann. Der Innendurchmesser der Hülse entspricht dem Durchmesser des Presslings, der in der Hülse angeordnet und aufgenommen werden kann.

Der Pressling wird an einem Ende der Hülse so angeordnet, dass die exponierte Oberfläche des Presslings bündig mit einer Stirnseite der Hülse und den daran angrenzenden Wandflächen des Gehäuses bzw. des Einsatzes abschließt. Durch die Verschlusseinrichtung wird das dem Pressling abgewandte Ende der Hülse dicht verschlossen, so dass rückseitig kein Lösungsmedium eindringen und mit dem Pressling in Kontakt kommen kann. Die Verschlusseinrichtung ist dabei zweckmäßigerweise so ausgestaltet, dass eine stempelförmige Ausformung der Verschlusseinrichtung in die Hülse hineinragt und flächenbündig mit einer rückseitigen Oberfläche des Presslings abschließt.

Es können verschiedene Hülsen und jeweils an die Hülsen angepasste Verschlusseinrichtungen vorgesehen sein, die bei einem gleichbleibenden Außendurchmesser der Hülsen jeweils unterschiedliche Innendurchmesser aufweisen. Da die exponierte Oberfläche des Presslings, der in der Hülse aufgenommen ist, mit zunehmendem Innendurchmesser der Hülse ebenfalls zunimmt, können Presslinge mit unterschiedlich großer exponierter Oberfläche in demselben Gehäuse angeordnet und mit dem Einsatz in die Messzelle eingebracht werden. Da das Gehäuse unabhängig von der Größe des Presslings für jede Messung verwendet werden kann und lediglich die in dem Gehäuse angeordnete Hülse zusammen mit der Verschlusseinrichtung ausgewechselt werden müssen, lassen sich auf diese Weise zuverlässig und schnell präzise Messungen mit unterschiedlich großen exponierten Oberflächen von Presslingen durchführen, die auf Grund der gleichbleibenden Gehäusegeometrie unmittelbar miteinander verglichen werden können.

Ein derartiger Einsatz, der in einfacher Weise in die bereits bekannten und weithin verwendeten Durchfluss-Messzellen eingebracht werden kann, ermöglicht die Verwendung von standardisierten und monografierten Durchfluss-Messzellen zur Messung der inneren Freisetzungsrate eines Wirkstoffs. Die bereits vorhandenen Messzellen können in einfacher Weise umgerüstet und dann auch zur Messung der inneren Freisetzungsrate verwendet werden. Da derartige Durchfluss-Messzellen oftmals bereits vorhanden sind und deren Handhabung ebenfalls standardisiert und bekannt ist, können mit einfachen Mitteln zuverlässige und reproduzierbare Messwerte erzeugt werden.

Die Verwendung einer Durchfluss-Messzelle hat darüber hinaus den großen Vorteil, dass das aus der Messzelle abgeführte Lösungsmedium kontinuierlich einer Messeinrichtung zugeführt und analysiert werden kann, sodass nicht nur kumulative Messungen der nach Ablauf der Messdauer insgesamt erfolgten Freisetzung, sondern auch kontinuierliche bzw. differenzielle Messungen der inneren Freisetzungsrate möglich sind.

Es ist beispielsweise auch möglich, in kurzen Zeitabständen das jeweils verwendete Lösungsmedium zu wechseln, so dass im Verlauf eines Messvorgangs Messwerte zu verschiedenen Lösungsmedien erhalten werden können.

Der Einsatz kann so ausgestaltet sein, dass der Einsatz schnell und zuverlässig in die Messzelle eingebracht und dort an einer exakt vorgegebenen Position angeordnet bzw. befestigt werden kann. Ein anschließendes Herausnehmen des Einsatzes oder die Verwendung wechselnder Einsätze wird dadurch gleichermaßen erleichtert.

Vorzugsweise ist vorgesehen, dass die Ausnehmung zur Aufnahme des Presslings auf einer dem anströmenden Lösungsmedium zugewandten Seite des Einsatzes angeordnet ist. Eine derartige Anordnung und Ausrichtung der exponierten Oberfläche des Presslings begünstigt eine möglichst gleichmäßige Wechselwirkung der exponierten Oberfläche des Presslings mit dem Lösungsmedium. Auf diese Weise werden beispielsweise Abschattungseffekte durch den von dem Lösungsmedium umströmten Einsatz oder unerwünschte Turbulenzen, die sich gegebenenfalls benachbart zu einer strömungsabgewandten Seite des Einsatzes bilden können, von der exponierten Oberfläche des Presslings ferngehalten. Bei den aus der Praxis bekannten Durchflusszellen, bei denen das Lösungsmedium von unten nach oben durch die Messzelle gepumpt wird, befindet sich die Ausnehmung zur Aufnahme des Presslings zweckmäßigerweise an der Unterseite des Einsatzes, wobei die exponierte Oberfläche des Presslings im Wesentlichen horizontal ausgerichtet ist.

Gemäß einer vorteilhaften Ausgestaltung des Erfindungsgedankens ist vorgesehen, dass eine die Ausnehmung zur Aufnahme des Presslings umgebende Wandfläche des Einsatzes eine Neigung in Strömungsrichtung aufweist, so dass das anströmende Lösungsmedium seitlich von dem Pressling weggeführt wird. Die den Pressling umgebende, dem anströmenden Lösungsmedium zugewandte Seite des Einsatzes kann beispielsweise kegelstumpfförmig ausgestaltet sein. Das anströmende Lösungsmedium wird durch die neben dem Pressling schräg verlaufende Stirnfläche des Einsatzes seitlich von dem Pressling weg und um den Einsatz herum geführt. Auf Grund der sich ausbildenden Strömungsverhältnisse kann das Lösungsmedium nicht über einen längeren Zeitraum vor dem Pressling verweilen. Auf diese Weise wird kontinuierlich ein frisches Lösungsmedium an dem Pressling vorbeigeführt, so dass sich weder lokal unterschiedliche Konzentrationsverhältnisse noch eine unerwünschte Bläschenbildung in dem Bereich um den Pressling herum ausbilden können, welche möglicherweise die Messgenauigkeit beeinträchtigen könnten.

In vorteilhafter Weise ist vorgesehen, dass das Gehäuse sowie die Verschlusseinrichtung im Wesentlichen aus einem Kunststoffmaterial und die Hülse aus Metall bestehen. Der Pressling wird zweckmäßigerweise durch Komprimieren des Wirkstoffs in der Hülse hergestellt. Die hierfür erforderlichen Druckkräfte sind oftmals derart hoch, dass die Hülse zweckmäßigerweise aus einem ausreichend druckfesten Material wie beispielsweise Metall bestehen sollte, um gegebenenfalls mehrfach eine zuverlässige Herstellung eines Presslings zu ermöglichen, ohne dass eine unerwünschte Verformung oder gar eine Beschädigung der Hülse befürchtet werden muss.

Dagegen ist es vorteilhaft, wenn das Gehäuse sowie die Verschlusseinrichtung aus einem möglichst leichten Material wie beispielsweise aus einem inerten Kunststoffmaterial bestehen und auf diese Weise das Gesamtgewicht des Einsatzes zusammen mit dem darin aufgenommenen Pressling so gering wie möglich gehalten werden kann. Es hat sich gezeigt, dass bei einer geeigneten Formgebung und Materialwahl der einzelnen Bauteile des Einsatzes dessen Gesamtgewicht deutlich weniger als 200 g betragen kann und Gewichtsbestimmungen mit handelsüblichen Analysenwaagen möglich sind, die regelmäßig einen hoch präzisen, aber bis 210 g Maximalgewicht beschränkten Messbereich aufweisen.

Um auch während der Durchführung einer Messung eine optische Kontrolle und gegebenenfalls zusätzliche optische Messungen durchführen zu können ist es bevorzugt vorgesehen, dass das Gehäuse und die Verschlusseinrichtung aus einem weitgehend transparenten Kunststoffmaterial hergestellt sind.

Vorzugsweise ist vorgesehen, dass der Einsatz Strömungskanäle aufweist, durch welche das Lösungsmedium den Einsatz passieren kann. Die Strömungskanäle können dabei innenliegend beispielsweise als durchgehende Bohrungen ausgestaltet sein oder aber durch seitliche Ausnehmungen oder Nuten gebildet werden. Durch die Anzahl, die Anordnung und die jeweilige Ausgestaltung der Strömungskanäle kann ein gleichbleibender, weitgehend laminarer Strömungsverlauf des Lösungsmediums in der Umgebung des Einsatzes und damit im Bereich des Presslings begünstigt werden.

Die Abmessungen des Einsatzes sind dabei zweckmäßigerweise an die Abmessungen des Messraums der Messzelle angepasst, so dass der Einsatz schnell und zuverlässig an einer gleichbleibenden Stelle innerhalb des Messraums angeordnet werden kann und beispielsweise durch sein Eigengewicht oder kraftschlüssig während des Messvorgangs an der vorgegebenen Position zurückgehalten wird.

Nachfolgend wird ein Ausführungsbeispiel des Erfindungsgedankens näher erläutert, das in der Zeichnung dargestellt ist. Es zeigt:
Fig. 1 einen Einsatz für eine Messzelle in auseinander gezogener Darstellung,
Fig. 2 eine Schnittansicht des in Fig. 1 dargestellten Einsatzes ebenfalls in auseinander gezogener Darstellung,
Fig. 3 eine Schrägansicht einer Pressvorrichtung, die zur Aufnahme des Einsatzes und zur Herstellung eines Presslings geeignet ist und
Fig. 4 eine schematische Darstellung einer Durchfluss-Messzelle mit einem darin angeordneten Einsatz.

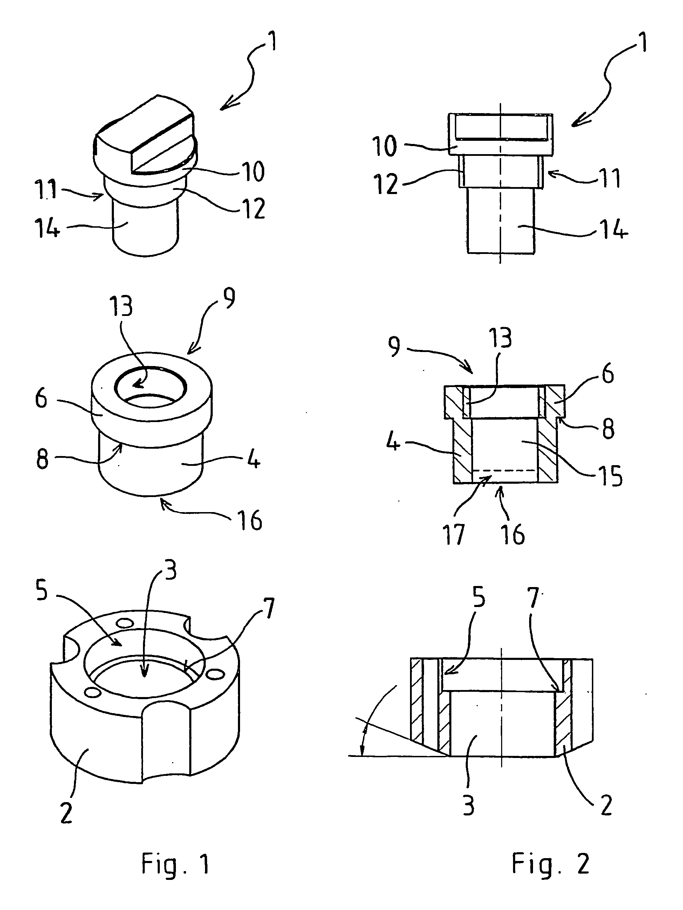

Ein in den Fig. 1 und 2 dargestellter Einsatz 1 weist ein im Wesentlichen hohlzylindrisches Gehäuse 2 mit einer mittig angeordneten Bohrung 3 auf. In der Bohrung 3 ist eine Hülse 4 angeordnet. Sowohl die Bohrung 3 als auch die Hülse 4 weisen jeweils an einem Ende eine konzentrisch ausgebildete, nach außen vorspringende ringförmige Ausnehmung 5 bzw. eine daran angepasste ringförmig vorspringende Ausformung 6 auf. Auf diese Weise werden einander zugewandte, ringförmige Anschlagflächen 7, 8 ausgebildet, mittels derer wiederholbar eine genaue relative Positionierung der Hülse 4 in der Bohrung 3 vorgegeben und gewährleistet werden kann.

Die Hülse 4 ist an einem strömungsabgewandten Ende 9 mittels einer schraubenförmigen Verschlusseinrichtung 10 dicht verschließbar. Die schraubenförmige Verschlusseinrichtung 10 weist einen Bereich 11 mit einem Außengewinde 12 auf, welches mit einem daran angepassten Innengewinde 13 an dem strömungsabgewandten Ende 9 der Hülse 4 in Eingriff bringbar ist, so dass die Verschlusseinrichtung 10 in die Hülse 4 eingeschraubt werden kann. Die Verschlusseinrichtung 10 weist in Verlängerung des Bereichs 11 mit dem Außengewinde 12 eine stempelförmige Ausformung 14 auf, deren Außendurchmesser an den Innendurchmesser der Hülse 4 angepasst ist. Die Verschlusseinrichtung 10 kann so weit in die Hülse 4 eingeschraubt werden, dass die stempelförmige Ausformung 14 einen Innenraum 15 in der Hülse 4 weitgehend ausfüllt und an einem strömungszugewandten Ende 16 der Hülse 4 lediglich ausreichend Platz zur Aufnahme eines in den Fig. 1 und 2 nicht dargestellten Presslings lässt. Bei einer in die Hülse 4 eingeschraubten Verschlusseinrichtung 10 wird durch die Hülse 4 und die stempelförmige Ausformung 14 eine Ausnehmung 17 zur Aufnahme eines Presslings gebildet.

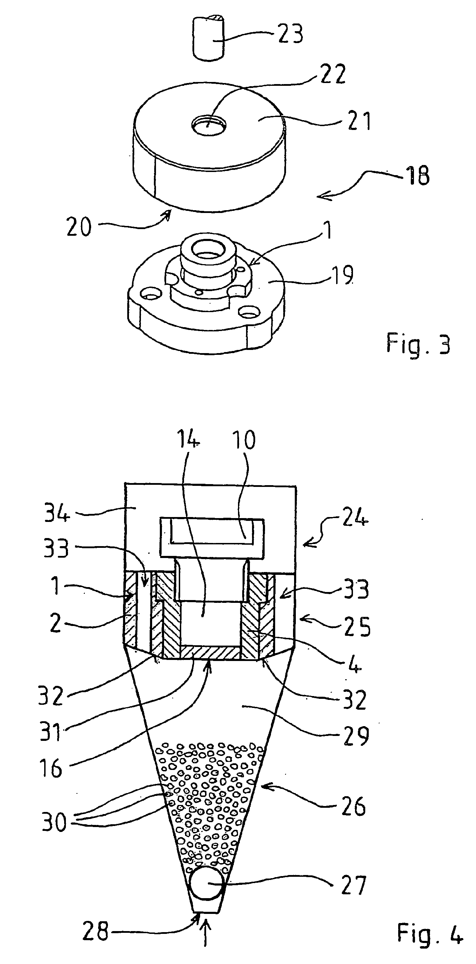

Durch Verwendung einer beispielhaft in Fig. 3 dargestellten Pressvorrichtung 18 kann in einfacher Weise unmittelbar in dem in Fig. 3 nicht vollständig dargestellten Einsatz 1 ein Pressling hergestellt werden. Die Pressvorrichtung 18 weist eine plane Grundplatte 19 auf. Ein Deckel 21 mit einer zur Aufnahme des Einsatzes 1 geeigneten Ausnehmung 20 kann mit der Grundplatte 19 mittels Schrauben oder anderer geeigneter Befestigungsmittel verbunden werden, so dass der Einsatz 1 zwischen der Grundplatte 19 und dem Deckel 21 angeordnet ist.

Der Deckel 21 weist eine mit der Hülse 4 in dem Einsatz 1 fluchtend angeordnete und an die Abmessungen des Innenraums 15 der Hülse 4 angepasste Bohrung 22 auf. Durch die Bohrung 22 hindurch kann ein Wirkstoff in den Innenraum 15 der Hülse 4 eingefüllt und anschließend mit einem durch Bohrung 22 in den Innenraum 15 der Hülse 4 einbringbaren Pressstempel 23 zu einem Pressling komprimiert werden.

Unmittelbar nach der Herstellung des Presslings können der Druckstempel 23 und der Deckel 21 entfernt und das offene Ende der Hülse 4 mit der schraubenförmigen Verschlusseinrichtung 10 verschlossen werden. Die stempelförmige Ausformung 14 der Verschlusseinrichtung 10 wird dabei in den Innenraum 15 der Hülse 4 eingeführt und die Verschlusseinrichtung 10 mit der Hülse 4 verschraubt, so dass die stempelförmige Ausformung 14 zu dem zuvor hergestellten Pressling hin abdichtet. Der Einsatz 1 kann anschließend entnommen und mit dem darin befindlichen Pressling in eine Messzelle eingebracht werden.

In Fig. 4 wird lediglich beispielhaft eine schematische Schnittansicht des vorangehend beschriebenen Einsatzes 1 in einer Messzelle 24 dargestellt. Bei der Messzelle 24 handelt es sich um eine Durchfluss-Messzelle mit einem im Wesentlichen hohlzylindrischen Abschnitt 25 und einem sich daran anschließenden, kegelförmig verjüngenden Befüllungsabschnitt 26. Durch eine üblicherweise mit einer Rubinglaskugel 27 gegen einen unerwünschten Rückfluss gesicherte Öffnung 28 kann ein in Fig. 4 nicht dargestelltes Lösungsmedium in einen Innenraum 29 der Messzelle 24 hineingepumpt werden. Das durch die unten angeordnete Öffnung 28 in den Innenraum 29 gepumpte Lösungsmedium wird anschließend durch eine Anzahl von Glasperlen 30 gedrückt, so dass sich weitgehend turbulenzfreie und im Wesentlichen laminare Strömungsverhältnisse des Lösungsmediums in dem Innenraum 29 der Messzelle 24 ausbilden. Der Einsatz 1 ist an einem der Öffnung 28 zugewandten Bereich des hohlzylindrischen Abschnitts 25 der Messzelle 24 angeordnet, wobei ein in der Hülse 4 aufgenommener Pressling 31 an dem strömungszugewandten Ende 16 des Einsatzes 1 in Richtung der Glasperlen 30 angeordnet ist. Das Lösungsmedium wird an dem Pressling 31 vorbeigeführt und an kegelstumpfförmig seitlich zurücktretenden Wandflächen 32 des Gehäuses 2 seitlich abgeführt und durch Strömungskanäle 33 an dem Einsatz 1 vorbeigeführt, um anschließend aus einem oberen Bereich 34 der Messzelle 24 entnommen und analysiert werden zu können.

## Patentansprüche

1. Vorrichtung zur Aufnahme eines Feststoffs in einer Durchfluss-Messzelle, bei welcher die Freisetzung des Feststoffs in einem durch die Durchfluss-Messzelle hindurchfließenden Lösungsmedium ermittelt werden kann, **dadurch gekennzeichnet, dass** die Vorrichtung einen in die Messzelle (24) einbringbaren Einsatz (1) mit einer Ausnehmung (17) zur Aufnahme eines Presslings (31) aufweist, wobei der Einsatz (1) ein Gehäuse (2) mit einer durchgehenden Bohrung (3), eine in der Bohrung (3) aufnehmbare Hülse (4) zur Aufnahme des Presslings (31) und eine Verschlusseinrichtung (10) des Presslings (31) und eine Verschlusseinrichtung (10) aufweist.

2. Vorrichtung nach Anspruch 1, **dadurch gekennzeichnet, dass** die Ausnehmung (17) zur Aufnahme des Presslings (31) an einer dem anströmenden Lösungsmedium zugewandten Seite (16) des Einsatzes (1) angeordnet ist.

3. Vorrichtung nach Anspruch 1 und/oder Anspruch 2, **dadurch gekennzeichnet, dass** eine die Ausnehmung (17) zur Aufnahme des Presslings (31) umgebende Wandfläche (32) des Einsatzes (1) eine Neigung in Strömungsrichtung aufweist, so dass das einströmende Lösungsmedium seitlich von dem Pressling (31) weggeführt wird.

4. Vorrichtung nach einem der vorangehenden Ansprüche, **dadurch gekennzeichnet, dass** das Gehäuse (2) sowie die Verschlusseinrichtung (10) im Wesentlichen aus einem Kunststoffmaterial und die Hülse (4) aus Metall bestehen.

5. Vorrichtung nach einem oder mehreren der vorangehenden Ansprüche, **dadurch gekennzeichnet, dass** der Einsatz (1) Strömungskanäle (33) aufweist, durch welche das Lösungsmedium den Einsatz (1) passieren kann.

6. Vorrichtung nach einem oder mehreren der vorangehenden Ansprüche, **dadurch gekennzeichnet, dass** die Abmessungen des Einsatzes (1) an die Abmessungen eines Messraums (29) der Messzelle (24) angepasst sind.

## Claims

1. Device for the accommodation of a solid in a through-flow measurement cell, in which the release of the solid can be determined in a solution medium flowing through the through-flow measurement cell, **characterised in that** the device has an insert (1) which can be introduced into the measurement cell (24), with a recess (17) for the accommodation of a compact (31), where the insert (1) has a housing (2) with a hole (3) through it, a sleeve (4), which can be accommodated in the hole (3), for the accommodation of the compact (31), and a closure device (10).

2. Device according to Claim 1, **characterised in that** the recess (17) for the accommodation of the compact (31) is arranged on a side (16) of the insert (1) facing the inflowing solution medium.

3. Device according to Claim 1 and/or Claim 2, **characterised in that** a wall surface (32) of the insert (1) which surrounds the recess (17) for the accommodation of the compact (31) has a tilt in the flow direction, so that the inflowing solution medium is transported laterally away from the compact (31).

4. Device according to one of the preceding claims, **characterised in that** the housing (2) and the closure device (10) essentially consist of a plastic material and the sleeve (4) consists of metal.

5. Device according to one or more of the preceding claims, **characterised in that** the insert (1) has flow channels (33) through which the solution medium can pass through the insert (1).

6. Device according to one or more of the preceding claims, **characterised in that** the dimensions of the insert (1) are matched to the dimensions of a measurement chamber (29) of the measurement cell (24).

## Revendications

1. Dispositif pour l'hébergement d'un solide dans une cellule de mesure à circulation traversante, dans lequel la libération du solide peut être déterminée dans un milieu de solution traversant en circulation la cellule de mesure à circulation traversante, **caractérisé en ce que** le dispositif comporte un insert (1) qui peut être introduit dans la cellule de mesure (24), avec un évidement (17) pour l'hébergement d'un matériau compact (31), où l'insert (1) comporte un logement (2) muni d'un trou (3) le traversant, une enveloppe (4), laquelle peut être logée dans le trou (3), pour l'hébergement du matériau compact (31), et un dispositif de fermeture (10).

2. Dispositif selon la revendication 1, **caractérisé en ce que** l'évidement (17) pour l'hébergement du matériau compact (31) est agencé sur un côté (16) de l'insert (1) faisant face au milieu de solution circulant en entrée.

3. Dispositif selon la revendication 1 et/ou la revendication 2, **caractérisé en ce qu'**une surface de paroi (32) de l'insert (1), laquelle entoure l'évidement (17) pour l'hébergement du matériau compact (31), présente une inclinaison dans la direction de circulation, de telle sorte que le milieu de solution circulant en entrée soit transporté latéralement à distance du matériau compact (31).

4. Dispositif selon l'une des revendications précédentes, **caractérisé en ce que** le logement (2) et le dispositif de fermeture (10) sont essentiellement constitués en une matière plastique et la gaine (4) est constituée en un métal.

5. Dispositif selon une ou plusieurs des revendications précédentes, **caractérisé en ce que** l'insert (1) comporte des canaux de circulation (33) par l'intermédiaire desquels le milieu de solution peut traverser l'insert (1).

6. Dispositif selon une ou plusieurs des revendications précédentes, **caractérisé en ce que** les dimensions de l'insert (1) sont en correspondance avec les dimensions d'une chambre de mesure (29) de la cellule de mesure (24).
